(19) Europäisches Patentamt
European Patent Office
Office européen des brevets

(11) **EP 3 033 043 B1**

(12) **EUROPEAN PATENT SPECIFICATION**

(45) Date of publication and mention
of the grant of the patent:
**25.10.2023 Bulletin 2023/43**

(21) Application number: **14753118.0**

(22) Date of filing: **12.08.2014**

(51) International Patent Classification (IPC):
***A61F 2/16*** *(2006.01)*

(52) Cooperative Patent Classification (CPC):
**A61F 2/1648; A61F 2/1613; A61F 2/1637;
A61F 2/164; A61F 2/1651;** A61F 2002/1683;
A61F 2002/1699

(86) International application number:
**PCT/GB2014/052458**

(87) International publication number:
**WO 2015/022514 (19.02.2015 Gazette 2015/07)**

(54) **INTRAOCULAR LENS SYSTEM**

KONTAKTLINSENSYSTEM

SYSTÈME DE LENTILLE INTRAOCULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2013 GB 201314428
20.03.2014 GB 201405005**

(43) Date of publication of application:
**22.06.2016 Bulletin 2016/25**

(73) Proprietor: **Syneos Health International Limited
Farnborough
Hampshire GU14 7BF (GB)**

(72) Inventors:
• **QURESHI, Muhammad Ali
London W1K 6SS (GB)**
• **ARTAL, Pablo
London
Greater London W1G 8GP (GB)**

• **SCOTT, Robbie
London
Greater London W1G 8GP (GB)**
• **TABERNERO, Juan
London
Greater London W1G 8GP (GB)**

(74) Representative: **Mewburn Ellis LLP
Aurora Building
Counterslip
Bristol BS1 6BX (GB)**

(56) References cited:
**WO-A1-2009/058755       WO-A1-2010/054255
US-A1- 2006 030 938       US-A1- 2006 100 704
US-A1- 2007 270 947       US-A1- 2009 210 054
US-A1- 2011 205 486       US-A1- 2012 136 438**

**Description**

**FIELD OF THE INVENTION**

The present invention relates to an intraocular lens system, particularly to an intraocular lens system comprising an anterior lens and a posterior lens.

**BACKGROUND OF THE INVENTION**

The most common condition affecting the macula is age-related macular degeneration (AMD) - this is also the most common cause of significant visual loss in the developed world. AMD results in loss of the light-sensitive cells (photoreceptors), and supporting tissue at the back of the eye, in a specialised part of the retina known as the macula. The condition most often involves the very central part of the macula (the fovea), an area which enables reading and the recognition of faces. In the majority of patients with age-related macular degeneration loss of vision occurs over a number of years and the pattern of visual loss allows for the maintenance of small islands of functioning photoreceptors in the macula. These remaining islands of tissue may permit sufferers to read but, because the density of light-sensitive cells reduces with increasing eccentricity from the fovea, visual resolution may be impaired such that at 3 degrees nasal to the central fovea, the visual acuity is reduced to 0.4 (compared with a visual acuity of 1.0 at 0 degrees), and at 5 degrees the visual acuity is further reduced to 0.34. Depending on the severity of the disease, patients may benefit from visual aids such as magnifying glasses, or the use of spectacle-mounted or hand-held telescopic devices that facilitate reading. Use of such devices is often restrictive because magnifying glasses are not easily portable (and require good lighting), and telescopic devices can severely reduce a patient's field of view. Despite the problems associated with reduced visual resolution, patients with age-related macular degeneration and similar conditions affecting the central visual field may still make effective use of residual macular tissue outside the damaged fovea (sometimes referred to as the 'preferred retinal locus' or PRL) although this may require the patient to learn to fixate eccentrically - something that is not always easily accomplished. One potential method of improving patients' fixation is to undertake surgery to introduce a device to modify the path of light in the eye such that images are focused on the PRL with or without a magnifying effect. However, the precise location of the PRL varies from patient-to-patient and accurate targeting of the PRL using such an approach is essential if a patient's vision is not to be made worse. Furthermore, as the disease progresses and remaining islands of functioning retina shrink in size, the location of the PRL can shift and it may become necessary to alter the path of light in the eye to take account of this.

Current surgical approaches to the management of poor vision in age-related macular degeneration include the implantation of telescopic lenses, in some cases not dissimilar to those employed for use in cataract surgery. Such lenses have the advantage of superior optics without the disadvantages associated with the use of external telescopic devices. Furthermore, telescopic devices may be configured in such a way as to provide a magnified image that is focused on an area of healthy macula eccentric to the fovea. Most existing designs for these intraocular devices adopt variations on a Galilean telescope system such that a diverging intraocular lens (IOL) is sited in the eye behind a converging IOL.

IOLs may be used in cataract surgery to replace the focusing power of the natural crystalline lens and may be employed in isolation to provide a fixed focal distance or in combination to restore a degree of accommodation (the ability to see objects in the distance and close-up). The most commonly used approach is to site a single lens in the posterior chamber of the eye (behind the iris) - IOLs implanted into the posterior chamber of the eye are disclosed in U.S. Pat. Nos. 3718870; 3866249; 3913148; 3925825; 4014049; 4041552; 4053953; and 4285072. So-called 'accommodative' IOLs are disclosed in US Pat. Nos. 4254509; 4,253,199; 4,409,691; 5,674,282; 4,842,601 and WO 2008/077795 - these typically harness the effects of the ciliary muscle, normally active in deforming the natural crystalline lens, to alter the focusing power of a single piece IOL or similar arrangement of two or more implants. These devices differ from intraocular Galilean telescopes in that they do not confer the ability to magnify an image or displace it from the foveal centre.

A basic paraxial approach to an intraocular Galilean telescope is as follows:

$$D = f_{obj} + f_{oc}$$
$$M = -\frac{f_{oc}}{f_{obj}}$$
$$\Longrightarrow$$
$$f_{obj} = D\frac{M}{M-1}$$
$$f_{oc} = D\frac{-1}{M-1}$$

D = distance between lenses (assumed thin lenses)

M = magnification

fobj = focal length of the objective lens

foc = focal length of the ocular lens

Galilean intraocular telescopes that employ a light-diverging IOL located in the posterior chamber of the eye and a light-converging lens in the anterior chamber of the eye are disclosed by Orzalesi et al. (Ophthalmology Volume 114 Issue 5; 2007) and in U.S. Pat. No. 20120136438 A1. These systems provide for magnification of an image and the deviation of light to target healthy parts of the macula. The latter is achieved by displacement of the diverging lens in a direction perpendicular to the optic axis of the converging lens by means of an asymmetrical haptic design (the haptic is the supporting arm of an IOL, most often seen in pairs with each one attached at opposite sides of the implant to ensure the position of the IOL in the eye remains stable). By shortening one haptic and lengthening the other it is possible to shift the diverging IOL such that a prismatic effect is achieved and light focused eccentric to fixation. The arrangement has a number of disadvantages. Firstly, the prismatic effect is conferred by the diverging IOL, which lies behind the converging IOL in both instances, thereby making access difficult for the purpose of rotating the diverging IOL to target the PRL should its location change at a future date. Secondly, the siting of an IOL in the anterior chamber is known to be associated with secondary pathology such as glaucoma and damage to the cornea of the eye. Thirdly, the optics of such a configuration are highly dependent on the IOLs remaining a fixed distance apart, for the purposes of magnification, and at a fixed displacement perpendicular to the optical axis (in the case of the diverging lens) for the purposes of targeting the PRL so that without consideration of the optimal configuration of the IOLs in relation to one another the system has the potential to make a patient's vision even worse.

Intraocular telescopes that take advantage of IOLs placed in fixed alignment are disclosed in U.S. Pat. Nos. 7186266; 6596026; 5391202; 7918886; 20040082995 and 20110153014. The principal disadvantages of fixing the diverging lens to the converging lens in these systems are that: 1) The arrangement may not permit the displacement of one lens in relation to the other to create the prismatic effect necessary to target the PRL (as is the case with most cylindrical one-piece designs); 2) in some instances, the prismatic effect, if achieved, may not be modifiable without replacing the implant; 3) in the case of systems where the device (or part of the device) is implanted in the capsular bag, fibrosis of the capsular bag over the implant may prevent its easy replacement or rotation should the need arise for adjustment in response to a change in the PRL; 4) the size of the implant is increased such that a larger incision in the eye is required to site it (this is associated with longer wound-healing time and increased astigmatism that may adversely affect the quality of vision). In addition the high dioptric power of the lenses employed requires careful consideration of the lens surfaces so as to optimise visual potential and avoid poor performance of the implant.

An intraocular lens system according to the preamble of claim 1 is known from the document US-A-2012/136438.

Consequently there exists the need for an intraocular telescope that may be sited through a small incision in the eye and one that provides the advantages of magnification and the ability to focus an image on the PRL, whilst also being flexible enough to allow for changes in the location of the patient's PRL and, critically, ensuring that any image is tightly focused at the back of the eye.

**SUMMARY OF INVENTION:**

According to the present invention there is provided an intraocular lens system comprising an anterior light-converging intraocular lens for positioning within the eye, the anterior lens having an anterior surface and a posterior surface; and a posterior light-diverging intraocular lens for positioning within the eye posterior to the anterior lens, the posterior lens having an anterior surface and a posterior surface; wherein the posterior lens is separate to the anterior lens; and wherein at least one of the surfaces of the anterior lens or surfaces of the posterior lens is a modified surface which includes a surface aberration which increases the depth of focus of the lens.

The surface aberration optimises image quality and may also provide for a magnified image over a range of retinal eccentricities.

The system of two or more intraocular lenses (IOLs) are arranged in the manner of a Galilean telescope to provide a magnified image at, or outside, the fovea in individuals with poor central vision.

The tolerance of the system is advantageously increased as a result of the aberration of the modified surface. This means that the relative positioning of the two lenses is less critical so the system is less sensitive to positioning requirements which arise for example due to the anatomy of the eye of the patient.

The system advantageously permits free rotation of the anterior IOL relative to the posterior IOL, as well as enabling its replacement if necessary. This lens configuration also ensures that the path of light can be easily modified should macular disease progress.

At least one optic (i.e at least one lens) has at least one aspherical surface. This optimizes the quality of the image presented to the retina of the eye. The remaining lens surfaces may be spherical or one or more may be rendered aspherical to further increase the tolerance of the system to errors in IOL positioning. All lens surfaces may be rendered aspherical to further increase the tolerance of the system to errors in IOL positioning.

Throughout this application, the terms "lens" and optic are used interchangeably. It should be understood that optic refers to a refractive component of the intraocular lens.

The use of two intraocular lenses (IOLs) for in concert provides a way to maximize the visual potential of patients with age-related macular degeneration and other progressive and non-progressive conditions that affect the macula and central field of vision.

The surface aberration includes a spherical aberration.

The modified surface is a rotationally symmetric polynomial surface. Optionally, the surface aberration includes an aberration of at least 4th order.

Optionally, the surface aberration includes an aberration of no more than 6th order.

Optionally, the surface sag (z coordinate) of the modified surface is given by:

$$z = \frac{cr^2}{1 + \sqrt{1 - (1+k)c^2 r^2}} + a_1 r^2 + a_2 r^4 + a_3 r^6$$

In this way, the aberration may be any high order aberration (particularly 4th to 6th order); a spherical aberration or otherwise such that the tolerance of the IOL system is improved. For example the system may induce a spherical aberration up to 0.4 microns of root mean squared for a 4mm pupil. The IOL system can therefore act to improve the patient's vision over a range of lens separation distances rather than at a specific separation distance. The IOL system avoids problems associated with other IOL systems where placement of the system in a patient's eye can actually result in a reduction in quality of the patient's vision due to optical effects associated with the relative locations of the two lenses.

In some embodiments the modified surface can be a simple conic surface with the surface sag (z) defined only by radius and conic constant.

Optionally, the modified surface includes a second aberration, the second aberration being a Zernike polynomial for any one of: tilt, defocus, astigmatism, or coma.

In this way, the IOL system is further optimised to correct for additional optical aberrations of specific patients.

Preferably, the intraocular lens system comprises a first and a second modified surface.

Preferably, both the anterior lens and the posterior lens each include a modified surface.

Preferably, the posterior surface of the anterior lens and the anterior surface of the posterior lens are both modified surfaces.

Preferably, the second modified surface includes a surface aberration which increases the depth of focus of the lens.

Preferably, the surface aberration of the second modified surface includes a spherical aberration.

Optionally, the second modified surface is a rotationally symmetric polynomial surface.

Optionally, the surface aberration of the second modified surface includes an aberration of at least 4th order.

Optionally, the surface aberration of the second modified surface includes an aberration of no more than 6th order.

Optionally, the surface sag (z coordinate) of the second modified surface is given by:

$$z = \frac{cr^3}{1+\sqrt{1-(1+k)c^2r^2}} + a_1 r^2 + a_2 r^4 + a_3 r^6$$

In this way, the aberration may be any high order aberration (particularly 4th to 6th order); a spherical aberration or otherwise.

In some embodiments the second modified surface can be a simple conic surface with the surface sag (z) defined only by radius and conic constant.

Optionally, the second modified surface includes a second aberration, the second aberration being a Zernike polynomial for any one of: tilt, defocus, astigmatism, or coma. This aberration may be at least a 4th order aberration. Optionally, it may be no more than a 6th order aberration.

In this way, the IOL system is further optimised to correct for additional optical aberrations of specific patients.

Optionally all four of the surfaces are modified surfaces to increase the range of depth of focus thereby increasing the tolerance of the positioning of the lenses in the eye.

Optionally, the intraocular lens system further includes: an anterior lens positioning means; and a posterior lens positioning means; wherein the anterior positioning means is configured such that when the anterior lens is positioned within the eye, the anterior positioning means locates the anterior lens so that it is aligned with the optical axis of the eye; and wherein the posterior positioning means is configured such that when the posterior lens is positioned within the eye, the posterior positioning means locates the posterior lens in so that it is aligned with the optical axis of the eye.

In this case, the anterior positioning means may comprise a plurality of haptics of equal lengths.

In this way, the haptics are capable of being configured in a symmetrical haptic design when in use such that the image focused onto the retina by the IOL system is focused at the fovea.

Optionally, the intraocular lens system further includes: an anterior lens positioning means; and a posterior lens positioning means; wherein the anterior positioning means is configured such that when the anterior lens is positioned within the eye, the anterior positioning means locates the anterior lens so that it is displaced from alignment with the optical axis of the eye; and wherein the posterior positioning means is configured such that when the posterior lens is positioned within the eye, the posterior positioning means locates the posterior lens in so that it is aligned with the optical axis of the eye.

Optionally, the displacement of the anterior lens is vertical displacement.

In this case, the anterior positioning means may comprise a plurality of haptics of different lengths. In this way, the haptics are capable of being configured in an asymmetrical haptic design when in use such that the anterior lens is displaced from the optical axis of the eye, resulting in an image being focused onto the retina by the IOL system at an area eccentric to the fovea.

Regardless of the positioning means of the anterior lens, the posterior positioning means may comprise a plurality of haptics of equal lengths. In this way, the haptics are capable of being configured in a symmetrical haptic design when in use such that the position of the image focused onto the retina by the IOL system is determined by the position of the anterior lens relative to the optical axis of the eye.

The displacement of the anterior lens is preferably transverse to the optical axis of the IOL.

Optionally, the optical axis of the first IOL may be displaced from that of the second IOL by means of an asymmetric haptic design with one haptic being shorter than the other. Optionally, this displacement is vertical displacement.

Optionally, the anterior lens and the anterior positioning means is a single-piece; and/or the posterior lens and the posterior positioning means is a single-piece. For example, each lens may be moulded to include its haptics.

Preferably, one or both of the intraocular lenses is tinted yellow and thereby configured such that the lens material absorbs light having wavelengths below 390nm.

In this way, the transverse chromatic aberration is reduced thereby optimizing the optics of the system.

Optionally, one or both of the anterior and posterior optics are modified to include an opaque rim. In this way, vignetting (produced by large pupil sizes or decentration) is prevented.

Optionally, optics of the lenses are modified such that a magnified image may be focused on the retina at a wide angle from the foveal centre.

Optionally, the surfaces of one or both lenses is modified to incorporate a Fresnel prism.

Optionally a third intraocular lens with a surface incorporating a diffractive property is locatable between the first and second lens to increase the depth of focus of the system.

Optionally, the anterior lens is suitable to be positioned in the anterior chamber of the eye and the posterior lens is suitable to be located in the ciliary sulcus of the eye. The anterior lens may have a diameter which is no more than 5mm. Optionally, the anterior lens may have a diameter of no less than 4mm and no more than 5mm.

Optionally, the anterior lens is suitable to be positioned in the ciliary sulcus of the eye and the posterior lens is suitable to be positioned in the capsular bag of the eye. Again, the anterior lens may have a diameter which is no more than 5mm. Optionally, the anterior lens may have a diameter of no less than 4mm and no more than 5mm.

Preferably at least part of a lens is made from a biocompatible material.

Preferably both lenses are made either partially or entirely from a biocompatible material.

The biocompatible material may be silicone or an acrylic. The material may be a rigid material such as polymethylmethacrylate but may be a softer acrylic which may have hydrophobic or hydrophilic properties.

Optionally, the anterior lens is supported by a support device with a haptic design which enables the optical axis of the anterior lens to be freely moved vertically in relation to that of the posterior lens so as to enable a retinal image to be focused either at the foveal centre or at a range of areas on the retina eccentric to the foveal centre. The haptic design may be symmetrical.

The anterior lens and support device may be marked such that alignment of the lens with a particular mark delivers a set degree of prismatic effect.

Optionally, the rim of the anterior lens is rendered opaque so as to prevent vignetting.

Optionally, where the anterior lens includes a rim, the rim of the lens is modified to include a holding means, and the points at which it makes contact with the supporting plates are modified to include corresponding holding means such that the rim of the lens is held in position without the need for friction at set points along its axis of movement.

Optionally, the posterior lens is supported by a support device with a haptic design which permits the optical axis of the posterior lens to be freely moved vertically in relation to that of the anterior lens so as to enable a retinal image to be focused either at the foveal centre or at a range of areas on the retina eccentric to the foveal centre. Again, the haptic design may be symmetrical.

Optionally, the intraocular lens system includes haptics for the anterior lens and/or posterior lens(es) which are angled to enable said lens(es) to be tilted in a variety of directions relative to the optical axis of the eye.

Optionally, the optic of the second IOL (the posterior lens) is supported by a device with a symmetrical haptic design which enables the optical axis of the posterior optic to be freely moved vertically in relation to that of the first IOL so as to enable a retinal image to be focused either at the foveal centre or an infinite range of angles away from the foveal centre.

According to a second aspect of the present invention, there is provided an intraocular lens system comprising: an anterior light-converging intraocular lens for positioning within the eye; and a posterior light-diverging intraocular lens for positioning within the eye; wherein the anterior lens comprises an opaque rim.

Optionally, the anterior lens includes an opaque annulus. This is particularly useful where the natural pupils of the patient are large as it prevents blurring of the retinal image that may otherwise occur as a result of light which travels around the outside of the lens.

Where the anterior lens is equipped with an opaque annulus, the annulus may have an inner diameter of between 5mm and 7mm. The annulus may be a separate feature which is connectable to the lens.

One embodiment of the present invention comprises two separate IOLs. The first is a light-converging lens shaped and sized for siting anteriorly to the second optic in the ciliary sulcus of the eye. The second is a posterior light-diverging lens shaped and sized for siting in the capsular bag. This embodiment is best employed with the IOLs sited in these positions but other embodiments allow for siting of the light-converging lens in the anterior chamber of the eye and the light-diverging IOL in the ciliary sulcus or both IOLs in the ciliary sulcus. Both IOLs are stabilized in their relative positions by means of haptics attached to or continuous with the optic of each lens and the configuration provides a magnified image in the manner of a Galilean telescope (in some instances this may be all that is required). However, in order to target a PRL, the haptics attached to the anterior, light-converging lens may be configured asymmetrically in such a way that one is shorter than the other. This permits displacement of the centre of the light-converging optic in a plane perpendicular to the optical axis of the light-diverging lens - thereby conferring a prismatic effect that allows light to be focused on an area of the macula eccentric to the fovea. The system allows for free rotation of the anterior IOL in the ciliary sulcus and targeting of other areas of the macula, located at the same angle relative the optic axis of the eye, during subsequent procedures. Furthermore the present invention permits the removal of the anterior IOL during subsequent procedures and its replacement with an IOL based on the same design but with a different dioptric power, or haptic lengths, such that light may be focused on a part of the macula located at a different angle or focal point relative to the optic axis.

The flexible nature of the present invention is made possible by optimization of the lens surfaces to correct for a range of optical aberrations. Optimisation of the IOL surfaces is required in the first instance because of the high dioptric powers of the optics, since these deviate from the thin lens paraxial formula described earlier. Furthermore, an optically unrefined IOL system with a prismatic effect generates significant amounts of astigmatism and coma thereby degrading the quality of the image presented to the patient's fovea or preferred retinal locus. Each IOL of the present invention therefore has at least one aspherical surface. This allows for a significant improvement in the quality of the image obtained - without it the system would not improve on the features of the existing systems described above, would confer a very limited benefit and would very likely make an individual's vision worse.

Flexibility is also afforded by the fact that the anterior lens is separate to the posterior lens and is rotatably movable relative to the posterior lens, i.e. that there is an absence of any coupling between the two lenses of the present invention (thereby permitting free rotation of the anterior IOL or its exchange for a different IOL). However the distance between the two lenses along the optic axis is also a critical factor in determining the quality of the retinal image - a small shift in the position of the lenses relative to one another along the optic axis results in the generation of significant refractive error and degrades the quality of the image presented to the macula. The current invention overcomes this problem by further manipulation of one of the two remaining spherical surfaces of the IOLs in the system, the manipulation being aspherization of one of the two remaining spherical surfaces thereby inducing spherical aberration to increase the depth of focus and assure both a high quality of retinal image and a significant range of positioning tolerance. The optics of the system are further refined to take account of the effect of the vertical decentration between both lenses in inducing transverse chromatic aberration - this is done by applying a yellow tint to one or both of the IOLs included in the kit. Other modifications to either or both IOLs are included in the disclosure for the present invention, these variously include refinements to the optics, such as to reduce vignetting with larger pupils, and changes that permit a wider application of the device. It is contemplated that the kit will include a range of IOLs of varying refractivepowers, surfaces and haptic configurations to permit targeting of PRLs in a wide variety of patients including those with conditions other than AMD and those with high refractive errors and astigmatism.

Description of Figures

Embodiments of the current invention will be illustrated with reference to the accompanying drawings of which:

Figure 1 is a diagrammatic cross-sectional view of an eye

Figure 2 is a diagrammatic cross-sectional view of an eye featuring an embodiment of the current invention as set out in the present disclosure

Figure 3 is a top view and side view of the anterior IOL featured as part of the systems illustrated in FIGS. 2 and 6.

Figure 4 a top view and side view of the posterior IOL featured as part of the systems illustrated in FIGS. 2 and 6

Figure 5 is a side view of the arrangement of the two IOLs with respect to one another as part of the system featured in FIG. 2

Figure 6 shows a comparison of image quality for a 'normal design' and the aspherized design of the present invention that provides for an increase in the tolerance of the system for varying distances between the two optics

Figure 7 is a side, oblique and top view of an embodiment of the anterior IOL

Figure 8 is a top view and side view of an embodiment of the anterior IOL featured in Figures 2, 3 and 5

Figure 9 is a top view and side view of an embodiment of the posterior IOL featured in Figures 2, 4 and 5

Figure. 10 is a diagrammatic cross-sectional view of an eye featuring an embodiment of the current invention as set out in the present disclosure

It is a key feature of the present invention that the surfaces of each IOL optic are modelled/configured to minimize optical aberration and to increase the tolerance of IOL positioning. The surface characteristics of the intraocular lenses used in the present invention may be described using Zernicke polynomials, these are a complete set of orthogonal polynomials defined on a unit circle which can be used to fit a wavefront or surface sag over a circular domain. They efficiently represent common errors such as coma and spherical aberration and are described according to the equation:

$$z(\rho,\theta) = \sum_{i=1}^{15} a_i Z_i$$

Where p and $\theta$ represent the normalized radius and the azimuth angle respectively and $a_i$ is the weighting coefficient for this term.

Table 1 shows the first 15 Zernike terms and the aberrations each term signifies.

Table 1

| i | $Z_1(\rho,\theta)$ | |
|---|---|---|
| 1 | 1 | Piston |
| 2 | $2\rho\cos\theta$ | Tilt x |
| 3 | $2\rho\sin\theta$ | Tilt y |
| 4 | $\sqrt{3}\,(2\rho^2 - 1)$ | Defocus |
| 5 | $\sqrt{6}\,(2\rho^2\sin2\theta)$ | Astigmatism 1st order (45°) |
| 6 | $\sqrt{6}\,(2\rho^2\cos2\theta)$ | Astigmatism 1st order (0°) |
| 7 | $\sqrt{8}\,(3\rho^3 - 2\rho)\sin\theta$ | Coma y |
| 8 | $\sqrt{8}\,(3\rho^3 - 2\rho)\cos\theta$ | Coma x |
| 9 | $\sqrt{8}\,(\rho^3\sin\theta)$ | Trifoil 30° |
| 10 | $\sqrt{8}\,(\rho^3\cos\theta)$ | Trifoil 0° Trifoil 0° |
| 11 | $\sqrt{5}\,(6\rho^4 - 6\rho^2 + 1)$ | Spherical aberration |
| 12 | $\sqrt{10}\,(4\rho^4 - 3\rho^2)\cos2\theta$ | Astigmatism 2nd order (0°) |
| 13 | $\sqrt{10}\,(4\rho^4 - 3\rho^2)\sin2\theta$ | Astigmatism 2nd order (45°) |
| 14 | $\sqrt{10}\,(4\rho^4\cos4\theta)$ | Tetrafoil 0° |
| 15 | $\sqrt{10}\,(\rho^4\sin4\theta)$ | Tetrafoil 22.5° |

For the purposes of promoting a full understanding of the principles of the present disclosure, reference will now be made to the Figures. No limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure.

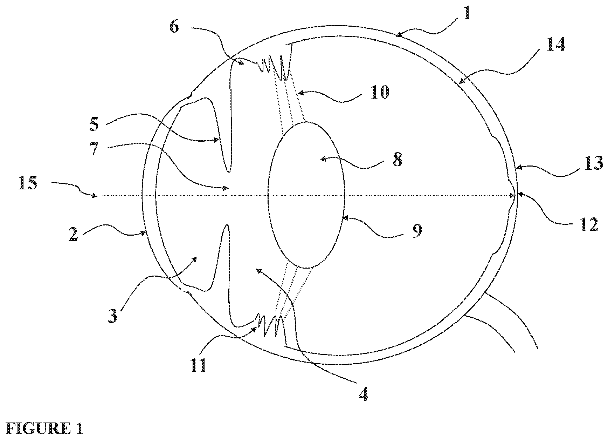

With reference to Figure 1, a representation of the human eye in cross-section. The eye is bounded by a tough fibrous coat, the sclera **1** which is absent anteriorly where it meets the cornea **2**. The cornea **2** is a transparent structure that provides the eye with most of its focusing power and forms the anterior boundary of the anterior chamber **3**. The posterior chamber **4** is separated from the anterior chamber **3** by the iris **5**. At the anterior periphery of the posterior chamber lies a depression known as the ciliary sulcus **6**. The iris **5** contains a round, central hole known as the pupil **7** that allows the passage of light to the natural crystalline lens **8**. The natural crystalline lens **8** is contained within a thin, continuous membrane known as the capsular bag **9** and attached to the capsular bag **9** are attached numerous fine ligaments known as the zonules **10**. At their peripheral extent the zonules **10** are attached to the ciliary muscle **11**. Changes in the shape of the natural crystalline lens **8** are made possible by the action of the ciliary muscle **11** and forces transmitted via the zonules **10** to the capsular bag **9** (an effect known as accommodation). The natural crystalline lens **8** acts to focus light rays on the fovea **12,** a highly specialised part of the macula **13** which in itself is a specialised part of the retina **14** (the light sensitive tissue at the back of the eye). The retina **14** consists of multiple layers that includes a light-sensitive layer of cells known as photoreceptors. The photoreceptors that facilitate colour vision and high-resolution vision (known as cones) are most highly concentrated at the macula **13** and, most particularly, at the fovea **12** - an area that is essential for reading and recognition of faces. It may be seen that damage to the fovea **12** and macula **13** may prevent light that has been focused at these sites from being detected with a consequent failure of any image being processed in the brain. Finally, the optical axis **15** is an imaginary line that defines the path along which light propagates through an optical system. For a system such as the eye the optical axis **15** passes through the center of curvature of the cornea **2** and natural crystalline lens **8** and coincides with the axis of rotational symmetry.

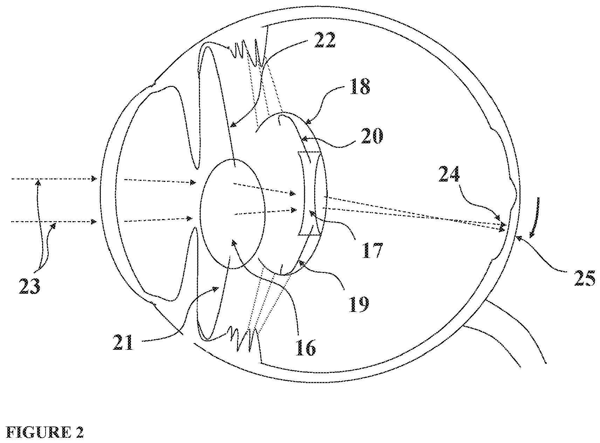

Referring now to both Figures 1 and 2. One embodiment of the present invention comprises an anterior light-converging IOL **16** located in the ciliary sulcus **6** and a posterior light-diverging IOL **17** located in the capsular bag **18**. It should be noted that in this embodiment the capsular bag **18** contains a circular defect anteriorly to facilitate removal of the natural crystalline lens or cataract in a manner consistent with current microincisional techniques employed during cataract surgery. The optical component of the anterior IOL **16** is maintained in position by haptics in an asymmetrical configuration such that the first haptic **21** is shorter than the second haptic **22** - the optical axis of the anterior lens therefore runs in a path parallel to that of the cornea. The optic of the posterior IOL is maintained in position in the capsular bag by means of haptics attached such that the first haptic **19** is the same length as the second **20** - the optical axis of the posterior lens therefore runs in line with that of the cornea. In this embodiment both optics **16, 17** are made of a hydrophobic material, such as soft acrylic polymer (refractive index 1.525; Abbe number 40; visible range transmission >95%; ultraviolet light transmission <0.5%), but generally the optics may be made from any transparent, biocompatible material used in intraocular lens construction, with calculations for optimisation of the optic surfaces (as set out below) revised accordingly. Similarly the haptics **19, 20, 21, 22** may be may be formed of any suitable polymeric material including polymethymethacrylate and/or polypropylene. The IOLs are designed to be foldable to facilitate implantation via a wound in the eye less than 5mm in length.

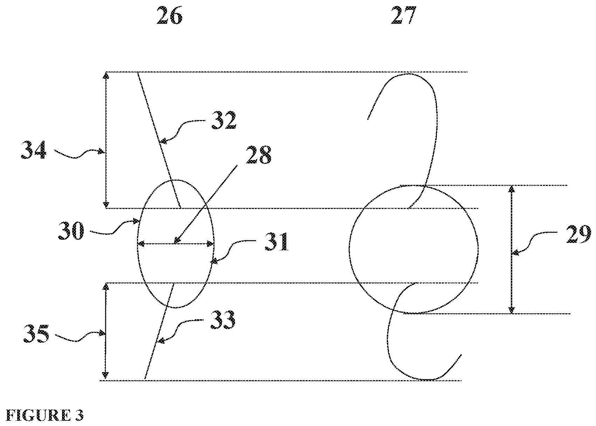

Referring to Figures 1, 2, 3, 4, 5 and 6, aspects of the IOLs and their arrangement are discussed in more detail. Figure 2 shows the anterior light-converging IOL in crosssection **26** and from the top **27**. The anterior converging IOL consists of a lightconverging optic of a thickness **28,** a diameter **29** and a dioptric power such that in conjunction with the posterior IOL, light may be focused on a region of the macula to provide a retinal image of a specific magnification. To achieve a retinal image of sufficient quality to benefit an individual with poor central vision, the optical design of the first lens is optimized such that it consists of a spherical anterior surface **30** and an aspheric posterior surface **31**. The anterior surface of the first lens is a standard spherical surface. The posterior surface of the first lens is a rotationally symmetric polynomial aspheric surface for which the surface sag (*z* co-ordinate) as a function of the radial coordinate *r* can be given by:

$$z = \frac{cr^2}{1 + \sqrt{1 - (1+k)c^2 r^2}} + a_1 r^2 + a_2 r^4 + a_3 r^6$$

Wherein,

   i) *c* is the inverse of the radius of curvature *R: c=1/R*
   ii) *k* is the conical constant (with a value ranging between -1 and 0
   iii) *a* is an aspheric polynomial coefficient, additional to the conical constant

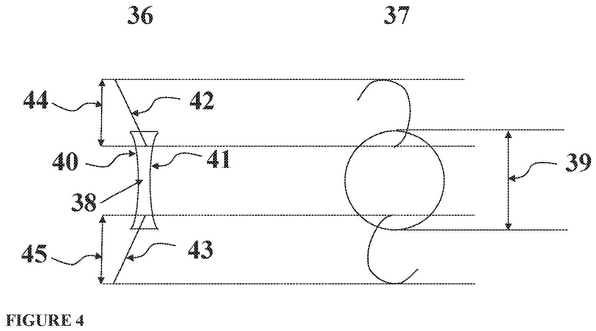

So that areas of macula at a set angle of deviation from the centre of the fovea may be targeted, two haptics are attached to or continuous with the anterior optic such that the first haptic **32** is longer than the second haptic **33**. The optic is therefore sited further from the point at which the haptic is designed to make contact with the eye **6** on one side **34** than on the other **35**. It should be noted that in this embodiment both haptics are angled anteriorly from the point at which they emerge from the optic in such a way that the optic is sited in a plane that lies posterior to that of the ciliary sulcus - in this way the anterior surface of the anterior IOL remains clear of the iris **5**. With reference to Figure 4, the posterior light-diverging IOL is shown in cross-section **36** and from the top **37**. The posterior light-diverging IOL consists of a light-diverging optic of a central thickness **38,** diameter **39** and dioptric power such that in conjunction with the anterior IOL, light may be focused on a region of the macula to provide a retinal image of specific magnification. Again, to achieve a retinal image of sufficient quality with this configuration the optical design of the posterior optic is optimised such that it consists of a rotationally symmetric polynomial aspheric anterior surface **40** and a standard conic posterior surface **41**. For the aspheric anterior surface **40,** the surface is a rotationally symmetric polynomial aspheric surface for which the surface sag z as a function of the radial coordinate *r* can be given by expression:

$$z = \frac{cr^2}{1+\sqrt{1-(1+k)c^2r^2}} + a_1 r^2 + a_2 r^4 + a_3 r^6$$

The sag (z coordinate) of the posterior surface of the second lens 41 can be given by the expression:

$$z = \frac{cr^2}{1+\sqrt{1-(1+k)c^2r^2}}$$

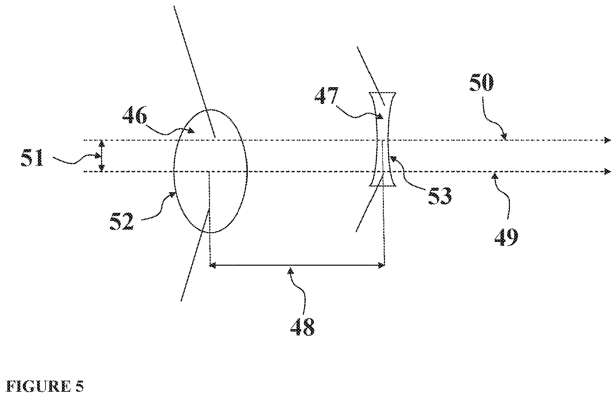

Attached to or continuous with the posterior optic are two haptics **42, 43** of equal length **44, 45.** The haptics are designed such that the posterior IOL may be sited in the capsular bag. It should be noted that in order to achieve a maximal distance from the anterior IOL it may be necessary to angle the haptics **42, 43** attached to the posterior IOL such that the optic lies in a plane posterior to the site where the haptics make contact with the periphery of the capsular bag **18.** With reference to Figure 5, that shows a cross-section of the arrangement of the anterior IOL in relation to the posterior IOL: The IOLs are arranged in the eye such that the anterior light-converging IOL **46** is sited at a fixed distance (in a direction parallel to the optical axes) from the posterior light-diverging IOL **47** resulting in a magnification of the retinal image of 1.2 to 1.4.

Since even a small deviation from the intended axial positioning of the two implants relative to one another could produce a significant refractive error and degradation of the image presented at the retina, the current invention increases the tolerance of the system for sub-optimal implant axial positioning by aspherizing one of the two remaining spherical surfaces in the system **52, 53** to add spherical aberration and increase the depth of focus. The precise amount of added spherical aberration is determined to assure both a good enough quality of retinal image and a significant range of positioning tolerance. This feature of the present invention ensures that it is capable of delivering a high quality of retinal image whilst accommodating variations in the practice of individual surgeons and alterations in the anatomy of the eye during the early and late post-operative periods.

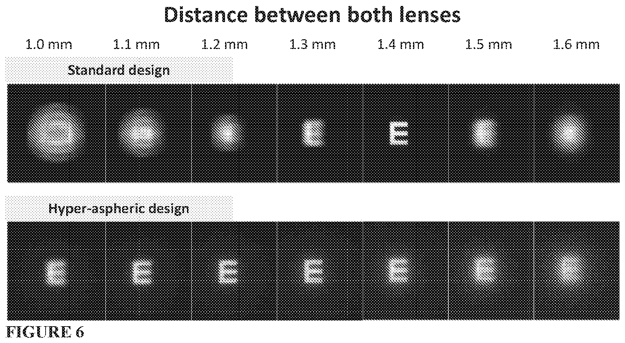

The benefits of added spherical aberration, in increasing the tolerance of IOL positioning in the present invention are shown in Figure 6.

The optics of the system are further optimised to take account of transverse chromatic aberration induced by the vertical displacement of the implants relative to one another **51,** this is achieved by adding a yellow tint to the implants during the manufacturing process. The addition of a yellow tint to the IOLs also confers the added benefit of macular protection from ultraviolet radiation.

With reference to Table 1, it can be seen that the surfaces of the optics of the IOLs of the present invention may be further optimised by the addition of values for Zernicke polynomials, besides those for spherical aberration. The surfaces may be expressed as a linear combination Zernicke polynomials including those for tilt, defocus, astigmatism, and coma, such that optical aberrations for individual patients are minimised. Consequent remodeling of the lenses means that at least one lens design parameter is changed - this may include the anterior surface shape and central radius and the posterior surface shape and central radius - and IOLs may be selected from a kit of lenses to achieve the desired effect.

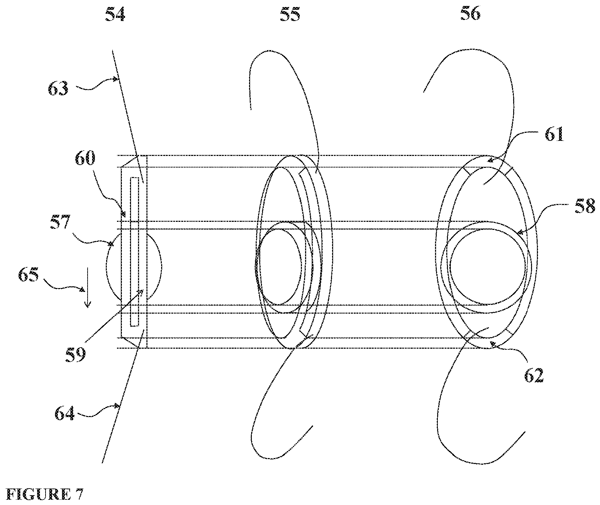

The asymmetric haptic lengths of the anterior light-converging IOL generate a vertical displacement 51 of its optic axis **49** from that of the light-diverging posterior IOL (and cornea) **50.** With reference to Figure 2, this displacement produces a deviation of path of light **24** such that a magnified retinal image is focused on an area of healthy macula **25** beyond the foveal centre. The materials, biomechanical properties, lengths and shapes of the haptics and the materials, surfaces, sizes and biomechanical properties of the anterior and posterior optics may be modified to achieve the desired displacement and corresponding retinal image (the haptics may form part of a single piece anterior or posterior IOL for example). It is contemplated that varying degrees of displacement may be achieved by selection of an anterior light-converging IOL of differing haptic lengths such that a range of angles extending from the centre of the fovea to >3 degrees (in the retinal plane), may be targeted, if necessary by using anterior segment imaging to ensure haptic lengths are appropriate for the size of an individual's ciliary sulcus or other location. Alternatively, Figure 7 shows an embodiment of the anterior light-diverging IOL shown from the side **54,** obliquely **55** and from the top **56.** In this embodiment the anterior light-converging optic **57** has an additional, optically neutral rim **58** (or an opaque rim to prevent vignetting) that permits the optic to be slid, with the application of force by the surgeon, between two plates made of biocompatible material **59, 60.** The plates **59, 60** are joined by supporting posts superiorly **61** and inferiorly **62** with, angled, symmetrical haptics **63, 64,** inserting into one of the two plates supporting the optic. This arrangement permits the optic to be slid from a neutral position vertically in a plane perpendicular to the optical axis as indicated by the arrow **65,** thereby conferring a range of prismatic effects without having to introduce IOLs with differing haptic lengths. The IOL may be marked in such a way that alignment of the optic with specific markings delivers a set amount of prismatic effect and deviation of the image from the foveal centre. Similarly the surface of the optic and its points of contact with the supporting plates may be modified such that the optic may held in set positions along its axis of movement. It is further contemplated that a range of anterior and corresponding posterior implants, consisting of a range of dioptric powers, optical surfaces, optic tints and haptic configurations may be included in the kit to facilitate targeting of the PRL in individual patients with a wide range of refractive errors (this includes toric optics to correct for high astigmatism). It will also be appreciated that rotation of the anterior light converging IOL around its optical axis, whilst keeping it in the same plane as the light-diverging posterior IOL, confers the ability to target a full range of locations in the macula at the set angle prescribed by the vertical displacement between the two IOLs (that is to say that the azimuth of the focal point of the system is fully adjustable). To facilitate this, an optically neutral mark may be included on the anterior light-converging optic so that its degree of rotation in relation to the eye can be determined without reference to the position of its haptics.

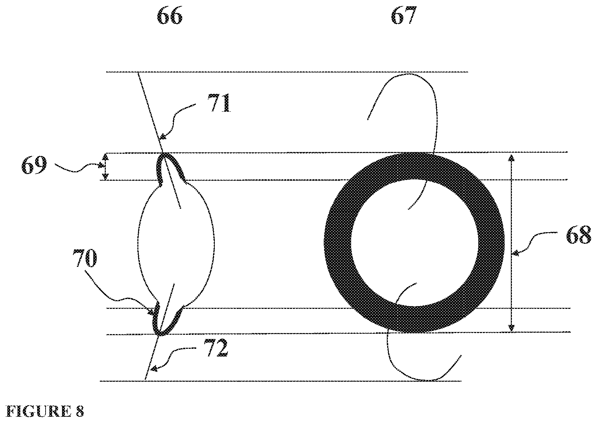

Referring now to Figure 8 which shows a version of the anterior light-converging IOL in cross-section **66** and from the top **67.** It is contemplated that with the current invention there is risk of visually significant vignetting occurring with larger pupil sizes, particularly where levels of vertical decentration between the anterior and posterior IOLs are high. A version of the anterior light-converging IOL designed to prevent such vignetting is shown **66, 67.** The diameter of the optic is increased in this embodiment **68** with an added rim **69** rendered opaque by the application of a biocompatible and stable opaque paint to its surface **70.**

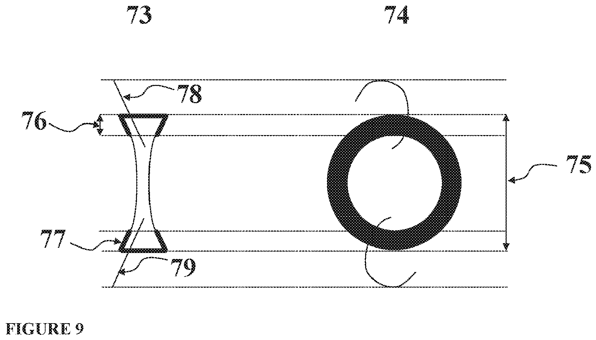

Alternatively an opaque, rim may be located on the surface of the optic, for example bonded to the optic as originally conceived to create the same effect. The rim is of sufficient width to prevent vignetting with larger pupils and/or at high levels of vertical displacement of the IOLs. The refractive part of the optic remains unaffected and the haptics **71, 72,** which may be of equal or differing lengths, insert into the optic as previously described. With reference to Figure 9, which shows a version of the posterior light-diverging IOL in cross-section **73** and from the top **74;** the same, or a similar, effect may be achieved by increasing the diameter of the posterior optic **75** to include a rim **76** that may be opaque and bonded to the optic or, as shown in the illustration, rendered opaque by means of a biocompatible and stable opaque paint applied to its surface **77** (the configuration of the haptics remaining unchanged **78, 79).**

In a further embodiment (not shown) the opaque rim may be located within part of the optic body.

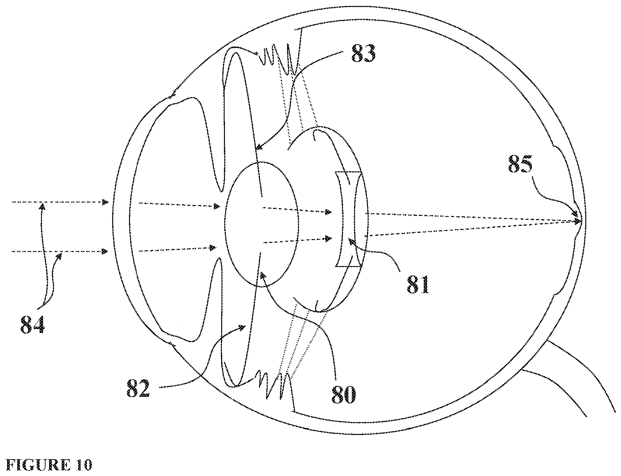

Reference to Figures 5 and 9 may now be made. Figure 10 shows a second embodiment of the current invention in a cross-section of the eye. This embodiment is the same as the first except that the vertical displacement of the IOLs relative to one another **51** is absent (that is to say that optical axes of the anterior **80** and posterior **81** optics are aligned with that of the cornea). To achieve this, the haptics of the first optic are of equal length **82, 83.** This focuses light entering the eye **84** in such a way that a magnified retinal image is centred on the fovea **85.** Alternatively the embodiment as shown in Figure 7 allows for positioning of the anterior optic such that its optical axis is in line with that of the posterior lens such that only magnification and no prismatic effect is generated. It is contemplated that in conditions such as amblyopia, where the overall structure of the fovea and macula remain intact, that it will not be necessary to deviate the path of light in the eye to target areas of the macula outside of the fovea. In this embodiment the haptics of the anterior light-converging IOL are of equal length and yellow tinting of the optics is not required in the absence of transverse chromatic aberration. It is further contemplated that a range of optics of varying dioptric powers, sizes and materials may be used, as in the first embodiment, to facilitate focusing of the magnified image on the fovea.

Although the invention is described in the preferred embodiments illustrated in the Figures attached, no restriction is intended by this. The design and configuration of the optical surfaces, including application of a tint to refine optical properties, are considered integral to the present invention and may be applied in a variety of circumstances. For example it is contemplated that an arrangement of the IOLs may include positioning of the anterior light-converging IOL in the anterior chamber 3 and the posterior light-diverging IOL in the ciliary sulcus 6 or both IOLs in the ciliary sulcus with revision of the optical surfaces, IOL dioptric powers and haptic designs accordingly. Similarly, it is contemplated that the asymmetric haptic design of the anterior light-converging IOL may be applied to the posterior light-diverging IOL so that the optics produce a combined prismatic effect.

Further embodiments (not shown) include the application of diffractive surfaces to one optic or both optics to permit a range of focal points in the eye (and consequently uncorrected distance and near vision); the application of a Fresnel prism to one or both optics to achieve a prismatic effect and targeting of the PRL - or the introduction of a third optic with one of the aforementioned characteristics, to either an anterior chamber, the ciliary sulcus or the capsular bag.

Again, whilst reference to use of the present invention in subjects with AMD is made, no restriction in terms of its use is intended. It is contemplated that the present invention will be used in a wide variety of clinical scenarios with or without displacement of the optics to achieve targeting of areas of the macula eccentric to fixation and with a range of magnification and refractive capabilities. The present invention is designed for insertion into the eye via a small (5mm) incision with or without use of a cartridge injector, an approach consistent with its use in the context of surgical techniques employed during natural crystalline lens or cataract extraction. As such it is expected that the present invention may be used in combination with natural crystalline lens extraction or at the time of cataract surgery or, if necessary, subsequent to cataract surgery/lens extraction (with its application - together with any necessary modifications to the optic surfaces, haptic design, optic materials and optic dioptric power - in addition to or instead of pre-existing implants in the eye).

In keeping with this approach, a range of monofocal IOLs may be provided that is designed for use in cases where the present invention is not indicated at initial surgery, but where the natural crystalline lens is removed and the patient wishes to retain the potential to use the present invention at a later date. Under these circumstances, the optics of the monofocal IOL implanted at the first operation will be optimised for use in conjunction with the present invention should this be required in the event that the patient develops a macular disease.

**Claims**

1. An intraocular lens system comprising:

    an anterior light-converging intraocular lens (16) for positioning within the eye, the anterior lens having an anterior surface and a posterior surface; and
    a posterior light-diverging intraocular lens (17) for positioning within the eye posterior to the anterior lens, the posterior lens having an anterior surface and a posterior surface;
    wherein the posterior lens is separate to the anterior lens; and
    the intraocular lens system being **characterized in that** at least one of the surfaces of the anterior lens or surfaces of the posterior lens is a modified surface which includes a surface spherical aberration which increases the depth of focus of the lens, wherein the modified surface is a rotationally symmetric polynomial surface.

2. An intraocular lens according to claim 1, wherein the surface aberration includes an aberration of at least $4^{th}$ order; and/or
   wherein the surface aberration includes an aberration of no more than $6^{th}$ order.

3. An intraocular lens according to any one of claims 1 or 2, wherein the surface sag (z coordinate) of the modified surface is given by:

$$z = \frac{cr^2}{1 + \sqrt{1 - (1+k)c^2r^2}} + \alpha_1 r^2 + \alpha_2 r^4 + \alpha_3 r^6$$

   wherein c is the inverse radius of curvature R and $c = \frac{1}{R}$ ; $k$ is the conical constant; and $\alpha_i$ where i is 1, 2, or 3 is an aspheric polynomial coefficient, and r is the radial coordinate.

4. The intraocular lens according to any one of the preceding claims, wherein the modified surface includes a second aberration, the second aberration being a Zernike polynomial for any one of:
   tilt, defocus, astigmatism, or coma.

5. An intraocular lens system according to any one of the preceding claims comprising a first and a second modified surface; and

    wherein optionally both the anterior lens (46) and the posterior lens (47) each include a modified surface; and
    wherein further optionally the posterior surface of the anterior lens and the anterior surface of the posterior lens are both modified surfaces.

6. An intraocular lens system according to any one of the preceding claims, further comprising:

    an anterior lens positioning means; and
    a posterior lens positioning means;
    wherein the anterior positioning means is configured such that when the anterior lens is positioned within the eye, the anterior positioning means locates the anterior lens (46) so that it is aligned with the optical axis of the eye; and
    wherein the posterior positioning means is configured such that when the posterior lens is positioned within the eye, the posterior positioning means locates the posterior lens (47) in so that it is aligned with the optical axis of the eye.

7. An intraocular lens system according to claim 6, wherein the anterior positioning means comprises a plurality of haptics (42, 43) of equal lengths.

8. An intraocular lens system according to any one of claims 1 to 7, further comprising:

an anterior lens positioning means; and
a posterior lens positioning means;
wherein the anterior positioning means is configured such that when the anterior lens is positioned within the eye, the anterior positioning means locates the anterior lens (57) so that it is displaced from alignment with the optical axis of the eye; and
wherein the posterior positioning means is configured such that when the posterior lens is positioned within the eye, the posterior positioning means locates the posterior lens in so that it is aligned with the optical axis of the eye.

9. An intraocular lens system according to claim 8, wherein the anterior positioning means comprises a plurality of haptics of different lengths.

10. An intraocular lens system according to any one of claims 6 to 9, wherein the anterior lens and the anterior positioning means is a single-piece; and/or wherein the posterior lens and the posterior positioning means is a single-piece.

11. An intraocular lens system according to any one of the preceding claims, wherein one or both of the intraocular lenses is tinted yellow.

12. An intraocular lens system of any one of the preceding claims wherein one or both of the anterior and posterior optics are modified to include an opaque rim (70).

13. An intraocular lens system of any one of the preceding claims, wherein optics of the lenses are modified such that a magnified image may be focused on the retina at a wide angle from the foveal centre.

14. An intraocular lens system according to any one of the preceding claims wherein the surfaces of one or both lenses is modified to incorporate a Fresnel prism.

15. An intraocular lens system according to any one of the preceding claims, wherein a third intraocular lens with a surface incorporating a Fresnel prism is locatable between the first and second lens to provide a prismatic effect.

16. An intraocular lens system according to any one of the preceding claims, wherein a third intraocular lens with a surface incorporating a diffractive property is locatable between the first and second lens to increase the depth of focus of the system.

17. An intraocular lens system according to any one of the preceding claims, where the anterior lens is suitable to be positioned in the anterior chamber of the eye and the posterior lens is suitable to be located in the ciliary sulcus of the eye.

18. An intraocular lens system according to any one of the preceding claims, wherein anterior lens is suitable to be positioned in the ciliary sulcus of the eye and the posterior lens is suitable to be positioned in the capsular bag of the eye.

19. An intraocular lens system according to any one of the preceding claims, wherein at least part of a lens is made from a biocompatible material; and
wherein optionally the biocompatible material is silicone or polymethylmethacrylate.

20. An intraocular lens system according to any one of claims 1 to claim 5, wherein the anterior lens is supported by a support device with a symmetrical haptic design which enables the optical axis of the anterior lens to be freely moved vertically in relation to that of the posterior lens so as to enable a retinal image to be focused either at the foveal centre or at a range of areas on the retina eccentric to the foveal centre.

21. An intraocular lens system according to claim 20, wherein the anterior lens and support device are marked such that alignment of the lens with a particular mark delivers a set degree of prismatic effect.

**22.** An intraocular lens system according to claim 20, wherein the anterior lens includes a rim; and wherein the rim of the lens is modified and the points at which it makes contact with the supporting plates are modified such that it is held in position without the need for friction at set points along its axis of movement.

**23.** An intraocular lens system according to any one of claims 1 to claim 5, wherein the posterior lens is supported by a support device with a symmetrical haptic design which permits the optical axis of the posterior lens to be freely moved vertically in relation to that of the anterior lens so as to enable a retinal image to be focused either at the foveal centre or at a range of areas on the retina eccentric to the foveal centre.

**24.** A system according to Claim 2 where the optic of the second IOL is supported by a device with a symmetrical haptic design which enables the optical axis of the posterior optic to be freely moved vertically in relation to that of the first IOL so as to enable a retinal image to be focused either at the foveal centre or an infinite range of angles away from the foveal centre.

**Patentansprüche**

**1.** Intraokulares Linsensystem, umfassend:

eine vordere intraokulare Lichtkonvexlinse (16) zum Positionieren innerhalb des Auges, wobei die vordere Linse eine vordere Fläche und eine hintere Fläche aufweist; und
eine hintere intraokulare Lichtkonkavlinse (17) zum Positionieren innerhalb des Auges hinter der vorderen Linse, wobei die hintere Linse eine vordere Fläche und eine hintere Fläche aufweist;
wobei die hintere Linse von der vorderen Linse getrennt ist; und
wobei das intraokulare Linsensystem **dadurch gekennzeichnet ist, dass** zumindest eine der Flächen der vorderen Linse oder Flächen der hinteren Linse eine modifizierte Fläche ist, die eine sphärische Oberflächenabberation umfasst, die die Tiefenschärfe der Linse erhöht, wobei die modifizierte Fläche eine rotationssymmetrische Polynomfläche ist.

**2.** Intraokulare Linse nach Anspruch 1, wobei die Oberflächenabberation eine Abberation der zumindest 4. Ordnung umfasst; und/oder
wobei die Oberflächenabberation eine Abberation der höchstens 6. Ordnung umfasst.

**3.** Intraokulare Linse nach einem der Ansprüche 1 bis 2, wobei die Oberflächenabsenkung (z-Koordinate) der modifizierten Fläche wie folgt angegeben wird:

$$z = \frac{cr^2}{1 + \sqrt{1 - (1+k)c^2r^2}} + \alpha_1 r^2 + \alpha_2 r^4 + \alpha_3 r^6$$

worin c der inverse Radius der Krümmung R ist und $c = \frac{1}{R}$ ; $k$ die konische Konstante ist; und $\alpha_i$ worin i = 1, 2 oder 3 ist, ein asphärischer Polynomkoeffizient ist und r die radiale Koordinate ist.

**4.** Intraokulare Linse nach einem der vorangegangenen Ansprüche, wobei die modifizierte Fläche eine zweite Abberation umfasst, wobei die zweite Abberation ein Zernike-Polynom für ein beliebiges der Folgenden ist: Schrägstellung, Unschärfe, Astigmatismus oder Koma.

**5.** Intraokulares Linsensystem nach einem der vorangegangenen Ansprüche, umfassend eine erste und eine zweite modifizierte Fläche; und

wobei sowohl die vordere Linse (46) als auch die hintere Linse (47) gegebenenfalls jeweils eine modifizierte Fläche aufweisen; und
wobei die hintere Fläche der vorderen Linse und die vordere Fläche der hinteren Linse gegebenenfalls ferner beide modifizierte Flächen sind.

6. Intraokulares Linsensystem nach einem der vorangegangenen Ansprüche, ferner umfassend:

ein vorderes Linsen-Positionierungsmittel; und
ein hinteres Linsen-Positionierungsmittel;
wobei das vordere Positionierungsmittel so konfiguriert ist, dass, wenn die vordere Linse innerhalb des Auges positioniert ist, das vordere Positionierungsmittel die vordere Linse (46) derart positioniert, dass sie mit der optischen Achse des Auges fluchtend ausgerichtet ist; und
wobei das hintere Positionierungsmittel so konfiguriert ist, dass, wenn die hintere Linse innerhalb des Auges positioniert ist, das hintere Positionierungsmittel die hintere Linse (47) derart positioniert, dass sie mit der optischen Achse des Auges fluchtend ausgerichtet ist.

7. Intraokulares Linsensystem nach Anspruch 6, wobei das vordere Positionierungsmittel eine Vielzahl von Haptikelementen (42, 43) gleicher Länge umfasst.

8. Intraokulares Linsensystem nach einem der Ansprüche 1 bis 7, das ferner Folgendes umfasst:

ein vorderes Linsen-Positionierungsmittel; und
ein hinteres Linsen-Positionierungsmittel;
wobei das vordere Positionierungsmittel so konfiguriert ist, dass, wenn die vordere Linse innerhalb des Auges positioniert ist, das vordere Positionierungsmittel die vordere Linse (57) derart positioniert, dass sie gegenüber der fluchtenden Ausrichtung mit der optischen Achse des Auges versetzt ist; und
wobei das hintere Positionierungsmittel so konfiguriert ist, dass, wenn die hintere Linse innerhalb des Auges positioniert ist, das hintere Positionierungsmittel die hintere Linse derart positioniert, dass sie mit der optischen Achse des Auges fluchtend ausgerichtet ist.

9. Intraokulares Linsensystem nach Anspruch 8, wobei das vordere Positionierungsmittel eine Vielzahl von Haptikelementen verschiedener Länge umfasst.

10. Intraokulares Linsensystem nach einem der Ansprüche 6 bis 9, wobei die vordere Linse und das vordere Positionierungsmittel einstückig sind; und/oder wobei die hintere Linse und das hintere Positionierungsmittel einstückig sind.

11. Intraokulares Linsensystem nach einem der vorangegangenen Ansprüche, wobei eine oder beide der intraokularen Linsen gelb getönt sind.

12. Intraokulares Linsensystem nach einem der vorangegangenen Ansprüche, wobei eine oder beide der vorderen und hinteren Optiken modifiziert sind, um einen intransparenten Rand (70) aufzuweisen.

13. Intraokulares Linsensystem nach einem der vorangegangenen Ansprüche, wobei die Optik der Linsen so modifiziert sind, dass ein vergrößertes Bild in einem Weitwinkel vom fovealen Zentrum auf der Hornhaut fokussiert werden kann.

14. Intraokulares Linsensystem nach einem der vorangegangenen Ansprüche, wobei die Flächen einer oder beider Linsen modifiziert sind, um ein Fresnel-Prisma zu enthalten.

15. Intraokulares Linsensystem nach einem der vorangegangenen Ansprüche, wobei eine dritte intraokulare Linse mit einer Fläche, die ein Fresnel-Prisma enthält, zwischen der ersten und der zweiten Linse positionierbar ist, um einen Prisma-Effekt bereitzustellen.

16. Intraokulares Linsensystem nach einem der vorangegangenen Ansprüche, wobei eine dritte intraokulare Linse mit einer Fläche, die eine Brechungseigenschaft aufweist, zwischen der ersten und der zweiten Linse positionierbar ist, um die Tiefenschärfe des Systems zu erhöhen.

17. Intraokulares Linsensystem nach einem der vorangegangenen Ansprüche, wobei die vordere Linse geeignet ist, um in der vorderen Kammer des Auges positioniert zu werden, und die hintere Linse geeignet ist, um in dem Ziliarsulkus des Auges positioniert zu werden.

18. Intraokulares Linsensystem nach einem der vorangegangenen Ansprüche, wobei die vordere Linse geeignet ist, um in dem Ziliarsulkus des Auges positioniert zu werden und die hintere Linse geeignet ist, um im Kapselsack des Auges positioniert zu werden.

19. Intraokulares Linsensystem nach einem der vorangegangenen Ansprüche, wobei zumindest ein Teil der Linse aus einem biokompatiblen Material besteht; und
wobei das biokompatible Material gegebenenfalls Silikon oder Polymethylmethacrylat ist.

20. Intraokulares Linsensystem nach einem der Ansprüche 1 bis 5, wobei die vordere Linse von einer Trägervorrichtung mit symmetrischem haptischem Aufbau getragen wird, die es ermöglicht, dass die optische Achse der vorderen Linse vertikal in Bezug auf jene der hinteren Linse frei bewegt werden kann, sodass ermöglicht wird, dass ein Netzhautbild entweder im fovealen Zentrum oder in mehreren Bereichen auf der Netzhaut exzentrisch zum fovealen Zentrum fokussiert wird.

21. Intraokulares Linsensystem nach Anspruch 20, wobei die vordere Linse und die Trägervorrichtung so markiert sind, dass eine Ausrichtung der Linse mit einer bestimmten Markierung einen fixierten Grad eines Prisma-Effekts liefert.

22. Intraokulares Linsensystem nach Anspruch 20, wobei die vordere Linse einen Rand umfasst; und
wobei der Rand der Linse modifiziert ist und die Punkte, an denen er mit den Trägerplatten in Kontakt kommt, so modifiziert sind, dass er ohne Reibung an festgelegten Punkten entlang seiner Bewegungsachse in Position gehalten wird.

23. Intraokulares Linsensystem nach einem der Ansprüche 1 bis 5, wobei die hintere Linse von einer Trägervorrichtung mit symmetrischem haptischem Aufbau getragen wird, die es ermöglicht, dass die optische Achse der hinteren Linse vertikal in Bezug auf jene der vorderen Linse frei bewegt werden kann, sodass ermöglicht wird, dass ein Netzhautbild entweder im fovealen Zentrum oder in mehreren Bereichen der Netzhaut exzentrisch zum fovealen Zentrum fokussiert wird.

24. System nach Anspruch 2, wobei die Optik der zweiten IOL von einer Vorrichtung mit symmetrischem haptischem Aufbau getragen wird, die es ermöglicht, dass die optische Achse der hinteren Optik vertikal in Bezug auf jene der ersten IOL frei bewegt werden kann, sodass ermöglicht wird, dass ein Netzhautbild entweder im fovealen Zentrum oder in einer unendlichen Mehrzahl von Winkeln weg von dem fovealen Zentrum fokussiert wird.

**Revendications**

1. Système de lentille intraoculaire comprenant :

une lentille intraoculaire faisant converger la lumière antérieure (16) pour un positionnement à l'intérieur de l'œil, la lentille antérieure présentant une surface antérieure et une surface postérieure ; et
une lentille intraoculaire faisant diverger la lumière postérieure (17) pour un positionnement à l'intérieur de l'œil postérieur à la lentille antérieure, la lentille postérieure présentant une surface antérieure et une surface postérieure ;
dans lequel la lentille postérieure est séparée de la lentille antérieure ; et
le système de lentille intraoculaire étant **caractérisé en ce qu'**au moins une des surfaces de la lentille antérieure ou des surfaces de la lentille postérieure est une surface modifiée qui comprend une aberration sphérique de surface qui augmente la profondeur de foyer de la lentille, dans lequel la surface modifiée est une surface polynomiale à symétrie de rotation.

2. Lentille intraoculaire selon la revendication 1, dans laquelle l'aberration de surface comprend une aberration au moins de 4ème ordre ; et/ou
dans lequel l'aberration de surface comprend une aberration de pas plus du 6ème ordre.

3. Lentille intraoculaire selon l'une quelconque des revendications 1 ou 2, dans laquelle la déclivité de surface (coordonnée z) de la surface modifiée est donnée par :

$$z = \frac{cr^2}{1 + \sqrt{1 - (1+k)c^2 r^2}} + a_1 r^2 + a_2 r^4 + a_3 r^6$$

dans laquelle c est le rayon de courbure inverse R et $c = \frac{1}{R}$ ; k est la constante conique ; et $a_i$ où i est 1, 2 ou 3 est un coefficient polynomial asphérique, et r est la coordonnée radiale.

4. Lentille intraoculaire selon l'une quelconque des revendications précédentes, dans laquelle la surface modifiée comprend une seconde aberration, la seconde aberration étant un polynôme de Zernike pour un quelconque parmi : inclinaison, défocalisation, astigmatisme ou coma.

5. Système de lentille intraoculaire selon l'une quelconque des revendications précédentes, comprenant une première et une seconde surface modifiées ; et

  dans lequel facultativement à la fois la lentille antérieure (46) et la lentille postérieure (47) comprennent chacune une surface modifiée ; et
  dans lequel en outre facultativement la surface postérieure de la lentille antérieure et la surface antérieure de la lentille postérieure sont toutes deux des surfaces modifiées.

6. Système de lentille intraoculaire selon l'une quelconque des revendications précédentes, comprenant en outre :

  un moyen de positionnement de lentille antérieure ; et
  un moyen de positionnement de lentille postérieure ;
  dans lequel le moyen de positionnement antérieur est configuré de telle sorte que lorsque la lentille antérieure est positionnée à l'intérieur de l'œil, le moyen de positionnement antérieur localise la lentille antérieure (46) de telle sorte qu'elle est alignée avec l'axe optique de l'œil ; et
  dans lequel le moyen de positionnement postérieur est configuré de telle sorte que lorsque la lentille postérieure est positionnée à l'intérieur de l'œil, le moyen de positionnement postérieur localise la lentille postérieure (47) de telle sorte qu'elle soit alignée avec l'axe optique de l'œil.

7. Système de lentille intraoculaire selon la revendication 6, dans lequel le moyen de positionnement antérieur comprend une pluralité d'haptiques (42, 43) de longueurs égales.

8. Système de lentille intraoculaire selon l'une quelconque des revendications 1 à 7, comprenant en outre :

  un moyen de positionnement de lentille antérieure ; et
  un moyen de positionnement de lentille postérieure ;
  dans lequel le moyen de positionnement antérieur est configuré de telle sorte que lorsque la lentille antérieure est positionnée à l'intérieur de l'œil, le moyen de positionnement antérieur localise la lentille antérieure (57) de telle sorte qu'elle est déplacée à partir d'un alignement avec l'axe optique de l'œil ; et
  dans lequel le moyen de positionnement postérieur est configuré de telle sorte que lorsque la lentille postérieure est positionnée à l'intérieur de l'œil, le moyen de positionnement postérieur localise la lentille postérieure de telle sorte qu'elle soit alignée avec l'axe optique de l'œil.

9. Système de lentille intraoculaire selon la revendication 8, dans lequel le moyen de positionnement antérieur comprend une pluralité d'haptiques de longueurs différentes.

10. Système de lentille intraoculaire selon l'une quelconque des revendications 6 à 9, dans lequel la lentille antérieure et le moyen de positionnement antérieur sont d'une seule pièce ; et/ou dans lequel la lentille postérieure et le moyen de positionnement postérieur sont d'une seule pièce.

11. Système de lentille intraoculaire selon l'une quelconque des revendications précédentes, dans lequel l'une des lentilles intraoculaires ou les deux sont teintées en jaune.

12. Système de lentille intraoculaire selon l'une quelconque des revendications précédentes, dans lequel l'une des optiques antérieure et postérieure, ou les deux, sont modifiées pour inclure un rebord opaque (70).

13. Système de lentille intraoculaire selon l'une quelconque des revendications précédentes, dans lequel les optiques des lentilles sont modifiées de telle sorte qu'une image agrandie peut être focalisée sur la rétine à un grand angle à partir du centre fovéal.

14. Système de lentille intraoculaire selon l'une quelconque des revendications précédentes, dans lequel les surfaces de l'une ou des deux lentilles sont modifiées pour incorporer un prisme de Fresnel.

15. Système de lentille intraoculaire selon l'une quelconque des revendications précédentes, dans lequel une troisième lentille intraoculaire avec une surface incorporant un prisme de Fresnel peut être positionnée entre la première et la deuxième lentille pour fournir un effet prismatique.

16. Système de lentille intraoculaire selon l'une quelconque des revendications précédentes, dans lequel une troisième lentille intraoculaire avec une surface incorporant une propriété de diffraction peut être située entre la première et la deuxième lentille pour augmenter la profondeur de foyer du système.

17. Système de lentille intraoculaire selon l'une quelconque des revendications précédentes, dans lequel la lentille antérieure est adaptée pour être positionnée dans la chambre antérieure de l'œil et la lentille postérieure est adaptée pour être située dans le sillon ciliaire de l'œil.

18. Système de lentille intraoculaire selon l'une quelconque des revendications précédentes, dans lequel la lentille antérieure est appropriée pour être positionnée dans le sillon ciliaire de l'œil et la lentille postérieure est appropriée pour être positionnée dans le sac capsulaire de l'œil.

19. Système de lentille intraoculaire selon l'une quelconque des revendications précédentes, dans lequel au moins une partie d'une lentille est réalisée à partir d'un matériau biocompatible ; et
dans lequel facultativement le matériau biocompatible est du silicone ou du polyméthylméthacrylate.

20. Système de lentille intraoculaire selon l'une quelconque des revendications 1 à 5, dans lequel la lentille antérieure est supportée par un dispositif de support avec une conception d'haptiques symétriques qui permet à l'axe optique de la lentille antérieure d'être librement déplacé verticalement par rapport à celui de la lentille postérieure de manière à permettre à une image rétinienne d'être focalisée soit au niveau du centre fovéal soit au niveau d'une plage de zones sur la rétine excentrique par rapport au centre fovéal.

21. Système de lentille intraoculaire selon la revendication 20, dans lequel la lentille antérieure et le dispositif de support sont marqués de telle sorte que l'alignement de la lentille avec une marque particulière délivre un degré défini d'effet prismatique.

22. Système de lentille intraoculaire selon la revendication 20, dans lequel la lentille antérieure comprend un rebord ; et dans lequel le rebord de la lentille est modifié et les points au niveau desquels il entre en contact avec les plaques de support sont modifiés de telle sorte qu'il est maintenu en position sans nécessiter de frottement à des points de consigne le long de son axe de déplacement.

23. Système de lentille intraoculaire selon l'une quelconque des revendications 1 à 5, dans lequel la lentille postérieure est supportée par un dispositif de support avec une conception d'haptiques symétriques qui permet à l'axe optique de la lentille postérieure d'être librement déplacé verticalement par rapport à celui de la lentille antérieure de manière à permettre à une image rétinienne d'être focalisée soit au niveau du centre fovéal soit au niveau d'une plage de zones sur la rétine excentrique par rapport au centre fovéal.

24. Système selon la revendication 2, dans lequel l'optique de la deuxième LIO est supportée par un dispositif avec une conception haptique symétrique qui permet à l'axe optique de l'optique postérieure d'être librement déplacé verticalement par rapport à celui de la première LIO de manière à permettre à une image rétinienne d'être focalisée soit au niveau du centre fovéal, soit au niveau d'une plage infinie d'angles s'éloignant du centre fovéal.

FIGURE 1

FIGURE 2

FIGURE 3

FIGURE 4

FIGURE 5

## Distance between both lenses

FIGURE 6

FIGURE 7

FIGURE 8

FIGURE 9

FIGURE 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3718870 A
- US 3866249 A
- US 3913148 A
- US 3925825 A
- US 4014049 A
- US 4041552 A
- US 4053953 A
- US 4285072 A
- US 4254509 A
- US 4253199 A
- US 4409691 A
- US 5674282 A
- US 4842601 A
- WO 2008077795 A
- US 20120136438 A1
- US 7186266 B
- US 6596026 B
- US 5391202 A
- US 7918886 B
- US 20040082995 A
- US 20110153014 A
- US 2012136438 A

**Non-patent literature cited in the description**

- **ORZALESI et al.** *Ophthalmology,* 2007, vol. 114 (5